# EUROPEAN PATENT SPECIFICATION

(11) **EP 0 522 226 B1**
(45) Date of publication and mention of the grant of the patent: **10.09.1997**
(21) Application number: 91830581.4
(22) Date of filing: 20.12.1991
(51) Int. Cl.: A61K 31/505, A61K 9/08

(54) **Eyedrop solution for the treatment of ocular hypertension containing ketauserin**
Ketanserin enthaltende Augentropfen zur Behandlung von intraokularem Druck
Gouttes ophtalmologiques à base de kétansérine pour le traitement de l'hypertension oculaire

(30) Priority: 12.07.1991 IT RM910519
(43) Date of publication of application: 13.01.1993
(73) Proprietor: Costagliola, Ciro, I-80122 Napoli (IT)
(72) Inventor: Costagliola, Ciro, I-80122 Napoli (IT)
(74) Representative: Di Cerbo, Mario

(56) References cited:
- EP-A- 0 268 309
- WO-A-91/02527
- STN INTERNATIONAL INFORMATION SERVICES DATA BASE: CHEMICAL ABSTRACTS, vol. 106, no. 17, abstract no. 131570r, Columbus, Ohio, US; F.W. CHANG et al.: "Mechanism of the ocular hypotensive action of ketanserin"

## Description

The present invention relates to an eyedrop solution for the treatment of ocular hypertension containing, at least as one of the active ingredients, a hydrosoluble salt of ketanserin, for example ketanserin tartrate. It is known that the phenomenon of ocular hypertension can manifest itself at any age with an incidence of 0.5% of the total population of the Mediterranean races and of 1-2% of the total of the population of the Nordic races. This disease, if not treated in time and with the appropriate therapies inevitably leads to blindness. The state of progress of the research in the specific sector has led to the developments of topical therapies based on beta blocking adrenergic agents (beta blockers), pilocarpine and alpha blocking adrenergic agents (alpha blockers) or of systemic therapies with the use of carbonic anhydrase inhibitors.

The adoption of these drugs has moreover revealed to be not completely satisfactory. The use of beta blockers has counterindications in patients suffering from cardio-respiratory diseases (atrio-ventricular block, asthma and so on) and furthermore it induces lacrimal hyposecretion with consequent dry eye symptoms. The alpha blocking adrenergic agents and pilocarpine, on their part, acting on the pupillary sphincters, induce miosis with a consequent reduction of the visual capacity and the dark adaptation of the patient. Furthermore, pilocarpine is responsable for pathological alteration of the stroma of the iris and its prolonged use may produce cataracts. The inhibitors of carbonic anhydrase have counterindications in patients with prostatic hypertrophy, liver and kidney alterations, due to electrolyte imbalances provoked by them.

In STN International Information Services data base: CA, vol. 106, n° 17, n° 131570r it is disclosed a topical solution for the eye based on ketanserin tartrate for lowering the ocular hypertension. However, taking it into account thant the human eye cornea does not contain Bowman membrane present in the cornea of the animals, and the fact that the pH of the ketanserint tartrate in the above document is about 1 and that the ketanserin hydrosoluble salts precipitate at neutral pH, the person skilled in the art would avoid to use such salts for human eyes.

It has now been unexpectedly found that all these inconveniences may be overcome by adopting the eyedrop solution of the present invention. This eyedrop solution also offers other advantages that will be apparent hereinafter. The eyedrop solution of the present invention is characterized by the fact that it essentially consists of the combination of the following components expressed in percent by weight:
- from 0.01 to 2.0% of at least a hydrosoluble salt of ketanserin;
- a buffer system biocompatible and pharmaceutically acceptable for maintaining the pH in the range 4.5 - 6.0;
- optionally, from 0.01 to 10% of at least a substance that may stabilize the lacrimal film;
- optionally, from 0.001 to 0.01% of a biocompatible and pharmaceutically acceptable preservative;
- optionally, a biocompatible and pharmaceutically acceptable colorimetric indicator, for revealing departures of the pH from the range 4.5 - 6.0;
- optionally, a solution that is isotonic with the osmotic pressure of the lacrimal fluid;
- optionally, at least one substance selected from the group comprising betablockers, pilocarpine, alpha blocking agents, inhibitors of carbonic anhydrase, inhibitors of the "angiotensin converting enzyme" (A.C.E.) and their combinations,
   the balance up to 100% being water.

Good results have been obtained when at least one of the hydrosoluble salts of ketanserin was ketanserin tartrate.

The substance for the stabilizing of the lacrimal film may be selected from the group comprising hydroxymethylcellulose, polyacrylic acid, propylenglycol, anhydrous dextrose and their combinations.

The preservative can be, for example, benzalkonium chloride.

The buffer system, biocompatible and pharmaceutically acceptable, for maintaining the pH of the eyedrop solution in the range 4.5 - 6.0 may consist of 0.1 - 1.0% of monopotassium phosphate and 0.1 - 1.5% of monopotassium phosphate and 0.1 to 1.5% of disodium phosphate.

In the eyedrop solution, in a concentration of up to 1.0% in weight, the free acid corresponding to the hydrosoluble salt of ketanserin utilized may also be present.

The mechanism of action of the eyedrop solution according to the invention permits a more coherent therapeutic strategy than that for example of beta blockers (which represent the most effective treatment currently commercially available) with regard to the pathological manifestations of the glaucomatous disease. In fact, as known, in this disease the outflow of the aqueous humor is altered, and it has been seen that, while beta blockers act prevalently on the formation of the aqueous humor the eyedrop solution according to the present invention acts by improving the outflow of the aqueous humor compromised by the disease.

Up to now, a general description of the present invention has been given. With the help of the following example, representing a specific embodiment of the invention, further details will now be provided so as to better clarify its objects, characteristics, advantages and modality of application.

### EXAMPLE

An eyedrop solution for the treatment of ocular hypertension according to the present invention has the following composition in percent by weight:
- ketanserin tartrate: 0.5%
- propylenglycol: 5.0%
- anhydrous dextrose: 5.0%
- tartaric acid: 0.1%,
the balance up to 100% of the composition being water.

The instillation of a drop (50 µl) of this eyedrop solution in the eye of a patient with chronic glaucome simplex produces a reduction in the ocular pressure from 25.3 to 20.7 mmHg after one hour. The obtained effect persists for approximately 12 hours. As a result a twice a day administration is sufficient.

The results obtained are comparable with those obtained using beta blockers that, amongst the active ingredients present in drugs currently available commercially, are the most effective for lowering the intraocular pressure. Moreover, the eyedrop solution according to the invention, contrarily to what happens for commercially available drugs containing beta blockers, can be administered to patients suffering from cardio-respiratory diseases and, in addition, does not induce a lacrimal hyposecretion with consequent dry eye symptoms in treated subjects.

## Claims

1. Eyedrop solution for the treatment of ocular hypertension, characterized by the fact that it essentially consists of the combination of the following components expressed in percent by weight:
- from 0.01 to 2.0% of at least a hydrosoluble salt of ketanserin;
- a biocompatible and pharmaceutically acceptable buffer system for maintaining the pH in the range 4.5 - 6.0;
- optionally, from 0.01 to 10% of at least one substance for stabilizing the lacrimal film;
- optionally, from 0.001 to 0.01% of a biocompatible and pharmaceutically acceptable preservative;
- optionally, a biocompatible and pharmaceutically acceptable colorimetric indicator for revealing departures of the pH from the range 4.5 - 6.0;
- optionally, a solution that is isotonic with the osmotic pressure of the lacrimal fluid;
- optionally, at least one substance selected from the group comprising beta blockers, pilocarpine, alpha blockers, inhibitors of carbonic anhydrase, inhibitors of the "angiotensin converting enzyme" and their combinations, the balance up to 100% being water.

2. Eyedrop solution for the treatment of ocular hypertension as per claim 1, in which the hydrosoluble salt of ketanserin is ketanserin tartrate.

3. Eyedrop solution for the treatment of ocular hypertension as per claims 1 or 2, in which the substance for the stabilization of the lacrimal film is selected from the group comprising hydroxymethyl cellulose, polyacrylic acid, propylenglycol, anhydrous dextrose and their combinations.

4. Eyedrop solution for the treatment of ocular hypertension as per any of the preceding claims, in which the preservative is benzalkonium chloride.

5. Eyedrop solution for the treatment of ocular hypertension as per any of the preceding claims, in which the biocompatible and pharmaceutically acceptable buffer system for maintaining the pH in the range 4.5 - 6.0 consists of 0.1 - 1.0% monopotassium phosphate and 0.1 - 1.5% disodium phosphate.

6. Eyedrop solution for the treatment of ocular hypertension as per any of the above claims from 1 to 5, which contains as a further component, at the expense of the water, up to 1% in weight of the free acid corresponding to the hydrosoluble salt of ketanserin used.

## Patentansprüche

1. Augentropfenlösung zur Behandlung von Augenüberdruck, **dadurch gekennzeichnet,** daß sie im wesentlichen aus der Kombination der folgenden Bestandteile, ausgedrückt in Gew.-%, besteht:
0,01 bis 2,0% von mindestens einem wasserlöslichen Salz von Ketanserin;
ein bioverträgliches und pharmazeutisch annehmbares Puffersystem zum Halten des pH-Wertes im Bereich 4,5 - 6,0;
gegebenenfalls 0,01 bis 10% von mindestens einer Substanz zum Stabilisieren des Tränenfilms;
gegebenenfalls 0,001 bis 0,01% von einem biovorträglichen und pharmazeutisch annehmbaren Konsorvierungsmittel;
gegebenenfalls ein bioverträglicher und pharmazeutisch annehmbarer colorimetrischer Indikator, um Abweichungen des pH-Werts vom Bereich 4,5 - 6,0 anzuzeigen;
gegebenenfalls eine Lösung, die mit dem osmotischen Druck der Tränenflüssigkeit isoton ist;
gegebenenfalls mindestens eine Substanz, ausgewählt aus der Gruppe umfassend Betablocker, Pilocarpin, Alphablocker, Carbonatdehydrase-Inhibitoren, Inhibitoren des "Angiotensin-Konversionsenzyms" und ihre Kombinationen, wobei der Rest auf 100% Wasser ist.

2. Augentropfenlösung zur Behandlung von Augenüberdruck nach Anspruch 1, worin das wasserlösliche Salz von Ketanserin Ketanserintartrat ist.

3. Aufgentropfenlösung zur Behandlung von Augenüberdruck nach den Ansprüchen 1 oder 2, worin die Substanz zur Stabilisierung des Tränenfilms ausgewählt ist aus der Gruppe umfassend Hydroxymethylcellulose, Polyacrylsäure, Propylenglycol, wasserfreie Dextrose und ihre Kombinationen.

4. Augentropfenlösung zur Behandlung von Augenüberdruck nach einem der vorhergehenden Ansprüche, worin das Konservierungsmittel Benzalkoniumchlorid ist.

5. Augentropfenlösung zur Behandlung von Augenüberdruck nach einem der vorhergehenden Ansprüche, worin das bioverträgliche und pharmazeutisch annehmbare Puffersystem zum Halten des pH-Werts im Bereich 4,5 - 6,0 aus 0,1 - 1,0% Monokaliumphosphat und 0,1 - 1,5% Dinatriumphosphat besteht.

6. Augentropfenlösung zur Behandlung von Augenüberdruck nach einem der vorstehenden Ansprüche 1 bis 5, welche als weiteren Bestandteil auf Kosten des Wassers bis zu 1 Gew.-% der freien Säure, die dem verwendeten wasserlöslichen Salz von Ketanserin entspricht, enthält.

## Revendications

1. Solution ophtalmologique en gouttes pour le traitement de l'hypertension oculaire, caractérisée par le fait qu'elle consiste essentiellement en la combinaison des composants suivants exprimés en pourcentage en poids :
- de 0,01 à 2,0 % d'au moins un sel hydrosoluble de cétansérine ;
- un système tampon biocompatible et pharmaceutiquement acceptable pour maintenir le pH dans la plage de 4,5 - 6,0 ;
- facultativement de 0,01 à 10 % d'au moins une substance pour stabiliser la pellicule lacrymale ;
- facultativement de 0,001 à 0,01 % d'un conservateur biocompatible et pharmaceutiquement acceptable ;
- facultativement, un indicateur colorimétrique biocompatible et pharmaceutiquement acceptable pour faire ressortir les écarts de pH de la plage 4,5 - 6,0 ;
- facultativement une solution qui est isotonique avec la pression osmotique du fluide lacrymal ;
- facultativement, au moins une substance choisie dans le groupe comprenant des bêtabloquants, la pilocarpine, des alphabloquants, des inhibiteurs de l'anhydrase carbonique, des inhibiteurs de "enzyme de conversion de l'angiotensine" et leurs combinaisons, la quantité suffisante pour atteindre 100 % étant de l'eau.

2. Solution ophtalmologique en gouttes pour le traitement de l'hypertension oculaire selon la revendication 1, dans laquelle le sel hydrosoluble de cétansérine est le tartrate de cétansérine.

3. Solution ophtalmologique en gouttes pour le traitement de l'hypertension oculaire selon la revendication 1 ou 2, dans laquelle la substance pour la stabilisation de la pellicule lacrymale est choisie dans le groupe comprenant la cellulose d'hydroxyméthyle, l'acide polyacrylique, le propylène-glycol, du dextrose anhydre et leurs combinaisons.

4. Solution ophtalmologique en gouttes pour le traitement de l'hypertension oculaire selon l'une quelconque des revendications précédentes, dans laquelle le conservateur est du chlorure de benzalconium.

5. Solution ophtalmologique en gouttes pour le traitement de l'hypertension oculaire selon l'une quelconque des revendications précédentes, dans laquelle le système tampon biocompatible et pharmaceutiquement acceptable pour maintenir le pH dans la plage de 4,5 - 6,0 consiste en 0,1 - 1,0 % de phosphate de monopotassium et 0,1 - 1,5 % de phosphate disodique.

6. Solution ophtalmologique en gouttes pour le traitement de l'hypertension oculaire selon l'une quelconque des revendications ci-dessus de 1 à 5, qui contient en tant que composant supplémentaire, aux dépens de l'eau, jusqu'à 1 % en poids de l'acide libre correspondant au sel hydrosoluble de la cétansérine utilisée.
